# EUROPÄISCHE PATENTSCHRIFT

(11) **EP 2 442 780 B1**
(45) Veröffentlichungstag und Bekanntmachung des Hinweises auf die Patenterteilung: **28.09.2016**
(21) Anmeldenummer: 10720925.6
(22) Anmeldetag: 26.05.2010
(51) Int. Cl.: A61K 8/19, A61K 8/25, A61K 8/26, A61K 8/28, A61K 8/365, A61Q 15/00

(54) **KOSMETISCHE ZUSAMMENSETZUNGEN MIT SILBERLACTAT**
COSMETIC COMPOSITIONS COMPRISING SILVER LACTATE
COMPOSITIONS COSMÉTIQUES CONTENANT DU LACTATE D'ARGENT

(30) Priorität: 19.06.2009 DE 102009027053
(43) Veröffentlichungstag der Anmeldung: 25.04.2012
(73) Patentinhaber: Henkel AG & Co. KGaA, 40589 Düsseldorf (DE)
(72) Erfinder: BANOWSKI, Bernhard, 40597 Düsseldorf (DE); BREUER, Imme, 40474 Düsseldorf (DE); HEIDE, Barbara, 47809 Krefeld (DE); TECKENBROCK, Gertraud, 45549 Sprockhövel (DE); ANDERHEGGEN, Bernd, 41189 Mönchengladbach (DE)
(86) Internationale Anmeldenummer: PCT/EP2010/057205
(87) Internationale Veröffentlichungsnummer: WO 2010/145919

(56) Entgegenhaltungen:
- WO-A2-2008/104310
- DE-U1-202008 014 407
- JP-A- 62 289 512
- US-B1- 6 444 726

## Beschreibung

Gegenstand der vorliegenden Anmeldung sind wasserfreie kosmetische oder dermatologische Zubereitungen, insbesondere Antitranspirantien, die Silberlactat enthalten.

Der Einsatz von Silber als Desinfektionsmittel in der Wundbehandlung, medizinischen Geräten sowie in pharmazeutischen und kosmetischen Zubereitungen ist im Stand der Technik bekannt. Für kosmetische Mittel ist beispielsweise ein Maximalgehalt von 4 % Silbernitrat erlaubt (KosmetikV, Anlage 3 zu § 3, Teil A).

Im Gegensatz zu wässrigen Lösungen löslicher Silbersalze (z. B. Silbernitrat oder Silberacetat) handelt es sich bei wässrigen kolloidalen Silberlösungen entweder um flüssige Dispersionen elementaren Silbers oder um flüssige Dispersionen komplexer schwerlöslicher Silberverbindungen. Die in Wasser dispergierbaren Ausgangsstoffe enthalten Silberionen dann nur in Spuren.

Es besteht für kosmetische Zubereitungen ein Bedarf an nicht-kolloidalem Silber.

Die WO 2006029213 A1 beschreibt antimikrobiell wirksame Silbercitratverbindungen. Diese in wässriger Citronensäure stabilisierten Silberverbindungen sind im Handel unter TINOSAN^{®} SDC erhältlich. Die Herstellung dieser Verbindungen über einen elektrochemischen Prozess wird in der EP 1041879 A1 beschrieben. TINOSAN^{®} SDC umfasst elektrolytisch erzeugte Silberionen (Ag⁺), und es liegt kein kolloidales Silber in der Mischung vor.

Das in den Druckschriften WO 2006029213 A1 und EP 1 041 879 A1 beschriebene TINOSAN^{®} SDC kann in kosmetischen, in wässriger und/oder emulsionsbasierter Zusammensetzung und mit verschiedenen Zusatzstoffen verwendet werden. Insbesondere werden in der WO 2006029213 A1 Deodorantien und Antitranspirantien offenbart, die TINOSAN^{®} SDC enthalten.

JP62289512 A offenbart Silbersalze von anorganischen und organischen Salzen, darunter Silberlactat, als Deodorantwirkstoffe.

WO 2008/104310A2 lehrt, dass Silber in Form von metallischem Silber, Silberverbindungen oder auf Trägermaterialien adsorbiertem Silber bzw. Silberverbindungen die bakterizide und fungizide Wirkung von Isothiazolinen synergistisch verstärkt. US 6444726 offenbart geträgerte Silberverbindungen als antimikrobiell wirksam.

Problematisch zeigt sich der Einsatz jedoch von in Wasser gelösten Silberverbindungen aufgrund von Korrosion bei metallischen Aufbewahrungsmitteln, wie beispielsweise Aerosoldosen. Dieser Effekt wird verstärkt durch die Gegenwart von die Korrosion fördernden Verbindungen, wie zum Beispiel saure Aluminium- und/oder Aluminium/Zirkoniumsalze. Im Aerosol kommt es bei Wasserkontakt außerdem häufig auch zu Verstopfung der Ventile sowie bei anderen kosmetischen Applikationsformen zu Verklumpung, Instabilitäten und Inhomogenitäten.

Ein weiteres Problem bei wasserhaltigen Mischungen kann bei der Verwendung von wasserlöslich verkapseltem Parfum auftreten. In Gegenwart von Wasser können diese verkapselten Parfüme quellen, verkleben und mit Auflösung reagieren.

Die DE 202008014407 U1 schlägt zur Lösung dieser Probleme vor, ein oder mehrere Verdickungsmittel, gewählt aus der Gruppe der Schichtsilikate und/oder Talkum, einzusetzen, die normalerweise mit einer geringen Menge an Wasser oder polarem Lösemittel geliert werden und gemäß DE 202008014407 U1 stattdessen mit Tinosan SDC und ggf. weiterem Wasser geliert werden. Auf diese Weise können Mittel bereitgestellt werden, die bis zu 10 Gew.-% Wasser (maximal 5 Gew.-% aus dem TINOSAN^{®} SDC plus weitere 5 Gew.-% aus übrigen Quellen) enthalten können.

Aus US 20050266081 A1 war ebenfalls bereits bekannt, die Gelbildung eines Schichtsilikats durch den Kontakt mit einer wässrigen Silbersalzlösung in Gegenwart eines Elektrolyten, wie Citronensäure, zu starten und so ein antibakteriell wirksames Gel herzustellen.

Es besteht weiter der Bedarf nach Möglichkeiten, Silber stabil in kosmetische Zusammensetzungen einzuarbeiten, auch wenn die Wassergehalte deutlich unterhalb von 10 Gew.-% liegen. Auch in wesentlich wasserärmeren Systemen, wo TINOSAN^{®} SDC versagt, sollte eine Darreichung von in Wasser gelösten antimikrobiell wirksamen Silberverbindungen aus eigentlich wasser-feindlicher Umgebung ermöglicht werden.

Gelöst werden die Probleme durch eine erfindungsgemäße wasserfreie kosmetische oder dermatologische Zubereitung, enthaltend Silberlactat in einer Gesamtmenge von 1,8 - 182 ppm, ein oder mehrere Verdickungsmittel, ausgewählt aus der Gruppe der Schichtsilikate und/oder Talkum, einen oder mehrere Antitranspirantwirkstoffe, Parfum und gegebenenfalls mindestens ein Treibmittel, wobei zur Ermittlung der Gesamtmenge gegebenenfalls in der Zubereitung enthaltenes Treibmittel nicht berücksichtigt wird.

Wasserfreie Zubereitung bedeutet erfindungsgemäß, dass zusätzliches Wasser bis zu einem Anteil von etwa 3 Gew.-%, bezogen auf die Gesamtmasse der Zubereitung, enthalten sein kann. Bevorzugt bedeutet wasserfrei einen Anteil von maximal 2 Gew.-%, insbesondere 0 Gew.-%, Wasser, bezogen auf die Gesamtmasse der Zubereitung.

Die Verdickungsmittel werden bevorzugt gewählt aus der Gruppe der Schichtsilikate und den Füllstoffen, wie Talkum. Schichtsilikate sind polymere kristalline Natriumdisilikate. Erfindungsgemäß bevorzugte Schichtsilikate werden gewählt aus Tonmineralien, wie Bentonit, Montmorrillonit, Nontronit, Hectorit, Saponit, Sauconit, Beidellit, Allevardit, Illit, Halloysit, Attapulgit und/oder Sepiolit. Bevorzugt ist Disteardimonium Hectorit. Hectorite sind M_{0,3}⁺(Mg_{2,7}Li_{0,3}) [Si₄O₁₀(OH)₂], M⁺ meist = Na⁺, zu den Smektiten gehörendes, dem Montmorillonit ähnliches, monoklines Tonmineral.

Ein weiteres erfindungsgemäßes Verdickungsmittel ist Talkum, Talk oder Talcum, bevorzugt Talcum mit der INCI-Bezeichnung Talc, E553b. Talkum ist ein natürliches, weit verbreitetes Magnesiumsilicat Mg₃[(OH)₂/Si₄O₁₀] oder 3 MgO · 4 SiO₂·H₂O, das zu den Dreischicht-(2:1-) Phyllosilicaten zählt, dessen dichtere Aggregate Speckstein heißen.

Die Verdickungsmittel, insbesondere Disteardimonium Hectorit oder Talkum, sind bevorzugt zu einem Anteil von 0,5 bis 10 Gew.-%, bevorzugt 1 - 7 Gew.-%, besonders bevorzugt 2 - 6 Gew.-%, außerordentlich bevorzugt 3 - 5 Gew.-%, enthalten, wobei zur Ermittlung des Anteils gegebenenfalls in der Zubereitung enthaltenes Treibmittel nicht berücksichtigt wird. Für Treibmittel-haltige Sprays beziehen sich diese Mengenangaben auf das Gewicht der Treibmittel-freien Zusammensetzung.

Kosmetische Antitranspirantien oder Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde. In so genannten Antitranspirantien (AT) kann durch Adstringentien - vorwiegend Aluminiumsalze, wie Aluminiumhydroxychlorid (Aluminiumchlorhydrat (ACH), das aktiviert sein kann (AACH)) oder Aluminium-Zirkonium-Salze (AZG) - die Bildung des Schweißes reduziert werden. Durch die Verwendung von antimikrobiellem Silberlactat in kosmetischen Antitranspirantien kann die Bakterienflora auf der Haut reduziert werden. Dabei werden die Geruch verursachenden Mikroorganismen wirksam reduziert. Der Schweißfluss selbst wird dadurch nicht beeinflusst, im Idealfall wird nur die mikrobielle Zersetzung des Schweißes zeitweilig gestoppt.

Die erfindungsgemäßen Zusammensetzungen enthalten mindestens einen Antitranspirant-Wirkstoff.

Bevorzugte Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze.

Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-oder Aluminiumzirkonium-Glycol-Komplexe, z. B. Aluminium- oder Aluminiumzirkonium-Propylenglycol-Komplexe, Aluminiumsesquichlorohydrex-PG oder Aluminiumsesquichlorohydrex-PEG, Aluminium-PG-dichlorohydrex oder Aluminium-PEG-dichlorohydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorohydrexglycin, Aluminiumzirconiumtetrachlorohydrexglycin, Aluminiumzirconiumpentachlorohydrexglycin, Aluminiumzirconiumoctachlorohydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirkoniumchlorohydrat).

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- oder Aluminium-Zirconiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze, wie sie beispielsweise in US 6902723 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(Al+Zr)-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt. Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein molares Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein molares Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen.

Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind. Diese Zirconiumsalze sind beispielsweise in der belgischen Schrift BE 825146 offenbart.

Weitere bevorzugte schweißhemmende Wirkstoffe sind in US 6663854 und US 20040009133 offenbart.

Die schweißhemmenden Wirkstoffe können sowohl in solubilisierter als auch in ungelöster, suspendierter Form vorliegen.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm und außerordentlich bevorzugt 10 - 15,5 µm, auf.

Bevorzugte Aluminiumsalze und Aluminiumzirconiumsalze weisen ein molares Metall-zu-Chlorid-Verhältnis von 0,9 - 2,0, bevorzugt 1,0 - 1,51, besonders bevorzugt 1,1 - 1,5, außerordentlich bevorzugt 1,3 - 1,4, auf.

Bevorzugte Aluminiumzirconiumchlorohydrate haben die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:CI = 1,1 - 0,9), und besonders bevorzugt m = 1,00 - 1,11 (entsprechend M:CI = 1,0 - 0,9), weiterhin sehr bevorzugt m = 1,02 - 1,11 (entsprechend M:CI = 0,98 - 0,9) sowie sehr bevorzugt m = 1,04 - 1,11 (entsprechend M:CI = 0,96 - 0,9).

Weitere bevorzugte Aluminiumzirconiumtrichlorohydrate haben die empirische Formel Al₄(OH)₁₀Cl₂ · Zr(OH)Cl.

Weitere bevorzugte Aluminiumzirconiumtetrachlorohydrate haben die empirische Formel Al₄(OH)₁₀Cl₂ · ZrCl₂.

Weitere bevorzugte Aluminiumzirconiumpentachlorohydrate haben die empirische Formel Al₈(OH)₂₀Cl₆ · Zr(OH)Cl.

Weitere bevorzugte Aluminiumzirconiumoctachlorohydrate haben die empirische Formel Al₈(OH)₁₈Cl₆ · Zr(OH)Cl.

Bei diesen Salzen ist im Allgemeinen etwas Hydratationswasser assoziativ gebunden, typischerweise 1 - 6 Mol Wasser pro Mol Salz, entsprechend 1 - 30 Gew.-%, bevorzugt 4 - 13 Gew.-% Hydratationswasser.

Bevorzugte schweißhemmende Salze sind Aluminium-Zirconiumtetrachlorohydrate (molares Verhältnis Al:Zr = 2-6; M:CI = 0.9-1.5, bevorzugt 0,95 : 1,3, besonders bevorzugt 1,0 : 1,1), insbesondere Salze mit einem molaren Metall-zu-Chlorid-Verhältnis von 0,9 - 1,1, bevorzugt 0,9 - 1,0. Weitere bevorzugte schweißhemmende Salze sind Aluminiumchlorohydrate mit einem molaren Metall-zu-Chlorid-Verhältnis von M:CI = 1,9 - 2,1.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Die vorstehend genannten Aluminium-Zirconiumtrichlorhydrate, Aluminium-Zirconiumtetrachlorhydrate, Aluminium-Zirconiumpentachlorhydrate und Aluminium-Zirconiumoctachlorhydrate liegen - sowohl aktiviert als auch nicht-aktiviert - bevorzugt als Komplex mit Glycin vor.

Besonders bevorzugte schweißhemmende Wirkstoffe sind ausgewählt aus aktiviertem Aluminium-Zirconiumtrichlorohydrex Glycine, insbesondere aktiviertem Aluminium-Zirconiumtrichlorohydrex Glycine mit einer kristallwasserfreien und Glycin-freien Aktivsubstanz (USP) von 69,5 - 88 Gew.-%, bevorzugt 72 - 85 Gew.-%, besonders bevorzugt 77 - 80 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8.

Weitere besonders bevorzugte schweißhemmende Wirkstoffe sind ausgewählt aus nicht-aktiviertem Aluminium-Zirconiumtrichlorohydrex Glycine, insbesondere nicht-aktiviertem Aluminium-Zirconiumtrichlorohydrex Glycine mit einer kristallwasserfreien und Glycin-freien Aktivsubstanz (USP) von 69,5 - 88 Gew.-%, bevorzugt 72 - 85 Gew.-%, besonders bevorzugt 77 - 80 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8.

Weitere besonders bevorzugte schweißhemmende Wirkstoffe sind ausgewählt aus aktiviertem Aluminium-Zirconiumtetrachlorohydrex Glycine, insbesondere aktiviertem Aluminium-Zirconiumtetrachlorohydrex Glycine mit einer kristallwasserfreien und Glycin-freien Aktivsubstanz (USP) von 72 - 88 Gew.-%, bevorzugt 77 - 85 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8.

Weitere besonders bevorzugte schweißhemmende Wirkstoffe sind ausgewählt aus nicht-aktiviertem Aluminium-Zirconiumtetrachlorohydrex Glycine, insbesondere nicht-aktiviertem Aluminium-Zirconiumtetrachlorohydrex Glycine mit einer kristallwasserfreien und Glycin-freien Aktivsubstanz (USP) von 72 - 88 Gew.-%, bevorzugt 77 - 85 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8.

Weitere besonders bevorzugte schweißhemmende Wirkstoffe sind ausgewählt aus aktiviertem Aluminium-Zirconiumpentachlorohydrex Glycine, insbesondere aktiviertem Aluminium-Zirconiumpentachlorohydrex Glycine mit einer kristallwasserfreien und Glycin-freien Aktivsubstanz (USP) von 72 - 88 Gew.-%, bevorzugt 77 - 86 Gew.-%, besonders bevorzugt 78 - 81,5 Gew.-%, jeweils bezogen auf den Rohstoff tel quel, einem molaren Metall:Cl-Verhältnis von 1,51 bis 2,0 und einem molaren Al:Zr-Verhältnis von 9,2 - 9,8.

Weitere besonders bevorzugte schweißhemmende Wirkstoffe sind ausgewählt aus nicht-aktiviertem Aluminium-Zirconiumpentachlorohydrex Glycine, insbesondere nicht-aktiviertem Aluminium-Zirconiumpentachlorohydrex Glycine mit einer kristallwasserfreien und Glycin-freien Aktivsubstanz (USP) von 72 - 88 Gew.-%, bevorzugt 77 - 86 Gew.-%, besonders bevorzugt 78 - 81,5 Gew.-%, jeweils bezogen auf den Rohstoff tel quel, einem molaren Metall:Cl-Verhältnis von 1,51 bis 2,0 und einem molaren Al:Zr-Verhältnis von 9,2 - 9,8.

Der Kristallwassergehalt beträgt bei den vorgenannten aktivierten wie nicht-aktivierten Aluminium-Zirconiumtrichlorohydrex Glycine, Aluminium-Zirconiumtetrachlorohydrex Glycine, Aluminium-Zirconiumpentachlorohydrex Glycine und Aluminium-Zirconiumoctachlorohydrex Glycine 1,5 - 20 Gew.-%, bevorzugt 7 - 15 Gew.-%, jeweils bezogen auf den Rohstoff tel quel.

Weitere bevorzugte Aluminium-Zirconiumtrichlorohydrex Glycine haben die empirische Formel [Al₄(OH)₁₀Cl₂ · Zr(OH)Cl] · NH₂CH₂COOH.

Weitere bevorzugte Aluminium-Zirconiumtetrachlorohydrex Glycine haben die empirische Formel [Al₄(OH)₁₀Cl₂ · ZrOCl₂] · NH₂CH₂COOH.

Weitere bevorzugte Aluminium-Zirconiumpentachlorohydrex Glycine haben die empirische Formel [Al₈(OH)₂₀Cl₄ · Zr(OH)Cl] · NH₂CH₂COOH.

Weitere bevorzugte Aluminium-Zirconiumoctachlorohydrex Glycine haben die empirische Formel [Al₈(OH)₁₈Cl₆ · Zr(OH)Cl] · NH₂CH₂COOH oder [Al₈(OH)₁₈Cl₆ · ZrOCl₂] · NH₂CH₂COOH.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5): 1 und ein molares Verhältnis von Betain zu Glycin von mindestens 0,001 : 1 auf. Entsprechende Verbindungen sind beispielsweise offenbart in US 7105691.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze mit einem hohen HPLC-Peak 5-Aluminium-Gehalt (auch als "E⁵AZCH" bezeichnet) sind beispielsweise offenbart in US 6436381 und US 6649152.

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt.

Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind beispielsweise in US 6923952 offenbart.

Weitere bevorzugte Antitranspirant-Wirkstoffe sind ausgewählt aus adstringierenden Titansalzen, wie sie beispielsweise in GB 2299506 A offenbart sind.

Die Antitranspirant-Wirkstoffe können als nicht-wässrige Lösungen oder als glycolische Solubilisate eingesetzt werden.

Besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 10 - 35 Gew.-%, besonders bevorzugt 11 - 28 Gew.-% und außerordentlich bevorzugt 12 - 20 Gew.-%, enthalten ist, bezogen auf das Gesamtgewicht der kristallwasserfreien und ligandenfreien Aktivsubstanz (USP) in der Gesamtzusammensetzung. Für Treibmittel-haltige Sprays beziehen sich diese Mengenangaben auf das Gewicht der Treibmittel-freien Zusammensetzung. Die Antitranspirant-Wirkstoffe werden in den erfindungsgemäßen Formulierungen bevorzugt in einer Menge von 1 bis 40 Gew.-%, besonders bevorzugt von 3 bis 15 Gew.-%, jeweils bezogen auf die Gesamtmasse der Zubereitung, d. h. inklusive ggf. vorhandener Treibgase, eingesetzt.

In Stickformulierungen liegt der Anteil an Antitranspirant-Wirkstoffen vorzugsweise im Bereich von 10 bis 25 Gew.-%., jeweils bezogen auf die Gesamtmasse der Zubereitung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit Reheis, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter der Bezeichnung Reach^{®} 301 wird ein Aluminiumsesquichlorohydrat von Reheis angeboten, das ebenfalls besonders bevorzugt ist.

Besonders bevorzugt sind Aluminium-Zirkonium-Tetrachlorohydrex-Glycin-Komplexe, die zum Beispiel von Summit Reheis unter der Bezeichnung Rezal^{®} 36 GP, Summit AZG-369 oder Summit AZG-364, oder, in aktivierter Qualität, als Summit Reach^{®} AZP-908, als Pulver im Handel sind.

Weiterhin besonders bevorzugt sind Aluminium-Zirkonium-Pentachlorohydrex-Glycin-Komplexe, die zum Beispiel in aktivierter Qualität von Summit unter den Bezeichnungen AAZG-3108 und AAZG-3110 als Pulver im Handel sind.

Auch vicinale Diole können als Antitranspirant-Wirkstoffe in den erfindungsgemäßen Zusammensetzungen enthalten sein. Bevorzugte vicinale Diole mit schweißhemmender Wirkung sind ausgewählt aus 1,2-Propylenglycol, Glycerin, 1,2-Butylenglycol, 1,2-Pentandiol, Dipropylenglycol, Tripropylenglycol, Diglycerin und Triglycerin. Die Gesamtmenge an vicinalen Diolen beträgt bevorzugt 10 bis 50 Gew.-%, besonderes bevorzugt 15%-30%, außerordentlich bevorzugt 15-25 Gew.-%, jeweils bezogen auf die Gesamtmasse der Zubereitung.

Optional können die erfindungsgemäßen Zusammensetzungen weitere Antitranspirant-Wirkstoffe und/oder Deodorant-Wirkstoffe enthalten.

Erfindungsgemäß bevorzugte Deodorant-Wirkstoffe, die zusätzlich zu Silberlactat enthalten sein können, sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe.

Bevorzugte Geruchsabsorber sind beispielsweise Zeolithe, Zinkricinoleat, Cyclodextrine, bestimmte Metalloxide, wie z. B. Aluminiumoxid, sowie Chlorophyll. Sie werden bevorzugt in einer Menge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, eingesetzt.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Linden-blütenöls) einsetzbar.

Weitere bevorzugte Deodorant-Wirkstoffe sind ausgewählt aus so genannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit einbezogen sind hier Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Weitere bevorzugte Deodorant-Wirkstoffe sind ausgewählt aus den keimhemmend wirkenden Parfümölen und den Deosafe^{®}-Parfümölen, die von der Firma Symrise, vormals Haarmann und Reimer, erhältlich sind.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugte erfindungsgemäße Deodorant- oder Antitranspirant-Zusammensetzungen sind dadurch gekennzeichnet, dass der mindestens eine Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether, Phenoxyethanol, keimhemmend wirkenden Parfümölen, Deosafe^{®}-Parfümölen (Deosafe^{®} ist ein eingetragenes Warenzeichen der Firma Symrise, vormals Haarmann & Reimer), präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen. Weitere bevorzugte erfindungsgemäße Zusammensetzungen sind dadurch gekennzeichnet, dass der mindestens eine Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%, jeweils bezogen auf das Gesamtgewicht der Aktivsubstanz des Deodorant-Wirkstoffs oder der Deodorant-Wirkstoffe in der Gesamtzusammensetzung, enthalten ist.

Silberlactat hat eine Molmasse von 196,94 g/mol. Silber hat eine Molmasse von 107,87 g/mol. Erfindungsgemäße Zusammensetzungen enthalten Silberlactat in einer Gesamtmenge von 1,8 - 182 ppm; dies entspricht einem Gehalt an Silber von 1 - 100 ppm, wobei zur Ermittlung der Gesamtmenge gegebenenfalls in der Zubereitung enthaltenes Treibmittel nicht berücksichtigt wird. Bevorzugte erfindungsgemäße Zusammensetzungen enthalten Silberlactat in einer Gesamtmenge von 3,6 - 91,3 ppm, bevorzugt 9,1 - 36,5 ppm, besonders bevorzugt 12,8 - 18,3 ppm, wobei zur Ermittlung der Gesamtmenge gegebenenfalls in der Zubereitung enthaltenes Treibmittel nicht berücksichtigt wird.

Weitere bevorzugte erfindungsgemäße Zusammensetzungen sind dadurch gekennzeichnet, dass Silber in einer Gesamtmenge von 2 - 50 ppm, bevorzugt 5 - 20 ppm, besonders bevorzugt 7 - 10 ppm, enthalten ist, wobei zur Ermittlung der Gesamtmenge gegebenenfalls in der Zubereitung enthaltenes Treibmittel nicht berücksichtigt wird.

Die Wirkstoffmischung ist gleich der Zubereitung, handelt es sich jedoch bei der Zubereitung um ein Aerosol, beziehen sich die Anteilsangaben auf die Wirkmischung ohne Treibgas. Die erfindungsgemäßen Aerosol-Zusammensetzungen werden bevorzugt so hergestellt, dass das Schichtsilikat mit einem Aktivator in einem Öl vorgeliert wird und dieses Gel mit einer Suspension aus dem Trägeröl, bevorzugt Cyclopentasiloxan, und einer Vormischung aus dem schweißhemmenden Wirkstoff und festem Silberlactat-Pulver vermischt wird. Anschließend wird die Suspension in eine Spraydose abgefüllt, die dann mit der gewünschten Menge an Treibgas beaufschlagt wird.

Die erfindungsgemäßen Stift-Zusammensetzungen werden bevorzugt so hergestellt, dass alle Trägerbestandteile, insbesondere die Öle, Wachse und Fettalkohole, geschmolzen und mit einer Vormischung aus Talkum oder Schichtsilikat, dem schweißhemmenden Wirkstoff und festem Silberlactat-Pulver vermischt und flüssig in geeignete Stifthülsen gegossen werden, worin sie zu schweißhemmenden Stiften erstarren.

Die erfindungsgemäßen Spray-Zubereitungen werden vorzugsweise dargereicht aus Standard Aerosol- und Stift-Verpackungen. Insbesondere sind sie für die Versprühung durch Standard Antitranspirant-Ventile und -Sprühköpfe geeignet, da sie Verstopfungsnachteile vermeiden helfen.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungs-mittel, Bakterizide, UV-Filter, Antioxidantien, Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Eine weitere bevorzugte Ausführungsform der Erfindung ist dadurch gekennzeichnet, dass die erfindungsgemäße oder erfindungsgemäß verwendete Zusammensetzung mindestens ein Monosaccharid mit 5 oder 6 Kohlenstoffatomen und/oder mindestens ein Disaccharid enthält. Eine besonders bevorzugte Ausführungsform der Erfindung ist dadurch gekennzeichnet, dass das Monosaccharid mit 5 oder 6 Kohlenstoffatomen ausgewählt ist aus Glucose, Fructose, Galactose, Arabinose, Ribose, Xylose, Lyxose, Allose, Altrose, Mannose, Gulose, Idose, Talose, Fucose und Rhamnose. Außerordentlich bevorzugt hiervon sind Glucose, Galactose, Fructose, Fucose und Rhamnose, insbesondere Glucose und Galactose.

Eine weitere besonders bevorzugte Ausführungsform der Erfindung ist dadurch gekennzeichnet, dass das Disaccharid ausgewählt ist aus Saccharose, Lactose und Trehalose. Außerordentlich bevorzugt hiervon sind Saccharose und Lactose, insbesondere Lactose.

Eine weitere besonders bevorzugte Ausführungsform der Erfindung ist dadurch gekennzeichnet, dass mindestens ein Monosaccharid mit 5 oder 6 Kohlenstoffatomen und/oder mindestens ein Disaccharid in einer Gesamtmenge von 0,001 bis 2,0 Gew.-%, bevorzugt 0,005 - 1,0 Gew.-%, besonders bevorzugt 0,01 - 0,5 Gew.-% und außerordentlich bevorzugt 0,03 - 0,1 Gew.-%, jeweils bezogen auf die gesamte Zusammensetzung, enthalten ist.

Sofern die erfindungsgemäßen Zusammensetzungen in Form eines Stiftes vorliegen, enthalten sie bevorzugt eine Lipid- oder Wachsmatrix, umfassend mindestens eine Lipid- oder Wachskomponente mit einem Schmelzpunkt > 50°C.

Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie der Antitranspirantzubereitung ausgezeichnete sensorische Eigenschaften und dem Stift insgesamt eine hohe Stabilität verleihen. Die Ester setzen sich aus gesättigten verzweigten oder unverzweigten Monocarbonsäuren und gesättigten verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten verzweigten oder unverzweigten Alkancarbonsäuren einer Kettenlänge von 12 bis 24 C-Atomen und den gesättigten verzweigten oder unverzweigten Alkoholen einer Kettenlänge von 16 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Kesterwachs bietet den Vorteil, dass es auch bei geringen Konzentrationen eine exzellente Ölgelierfähigkeit aufweist und so die Stiftmasse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C). Erfindungsgemäß ist als Wachskomponente hydriertes Rizinusöl, erhältlich z.B. als Handelsprodukt Cutina^{®} HR, besonders bevorzugt.

Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina^{®} FS 45 (Palmitin- und Stearinsäure).

Bevorzugte erfindungsgemäße Deodorant- oder Antitranspirant-Stifte sind dadurch gekennzeichnet, dass die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen. Besonders bevorzugte Lipid- oder Wachskomponenten-Mischungen a) sind ausgewählt aus Mischungen von Cetylbehenat, Stearylbehenat, gehärtetem Rizinusöl, Palmitinsäure und Stearinsäure. Weitere besonders bevorzugte Lipid- oder Wachskomponenten-Mischungen a) sind ausgewählt aus Mischungen von C₂₀-C₄₀-Alkylstearat, gehärtetem Rizinusöl, Palmitinsäure und Stearinsäure.

Weitere bevorzugte erfindungsgemäße Deodorant- oder Antitranspirant-Stifte sind dadurch gekennzeichnet, dass die Lipid- oder Wachskomponente/n insgesamt in Mengen von 4 - 20 Gew.-%, bevorzugt 8 - 15 Gew.-%, bezogen auf die Gesamtzusammensetzung, enthalten ist. In einer besonders bevorzugten Ausführungsform ist/sind der/die Ester aus einem gesättigten, einwertigen C₁₆-C₆₀-Alkohol und einer gesättigten C₈-C₃₆-Monocarbonsäure, der/die Lipid- oder Wachskomponente/n darstellt/ darstellen, in Mengen von insgesamt 2 - 10 Gew.-%, bevorzugt 2 - 6 Gew.-%, bezogen auf die Gesamtzusammensetzung, enthalten.

Bevorzugte erfindungsgemäße Zusammensetzungen, die als Spray oder Stift vorliegen, enthalten bevorzugt weiterhin mindestens ein bei 20 ° C flüssiges Öl, das keine Duftstoffkomponente und kein etherisches Öl darstellt. Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20) und 2-Ethylhexylalkohol. Weitere bevorzugte Ölkomponenten sind Mischungen aus Guerbetalkoholen und Guerbetalkoholestern, z.B. das Handelsprodukt Cetiol^{®} PGL (Hexyldecanol und Hexyldecyllaurat).

Weitere erfindungsgemäß bevorzugte Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte Öle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß bevorzugte Ölkomponenten sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere erfindungsgemäß bevorzugte Ölkomponenten sind ausgewählt aus den Anlagerungsprodukten von mindestens 6 Ethylenoxid und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57) und PPG-15-Stearylether (Arlamol^{®} E).

Weitere erfindungsgemäß bevorzugte Ölkomponenten sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß bevorzugte Ölkomponenten sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1.

Weitere erfindungsgemäß bevorzugte Ölkomponenten sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen. Es kann erfindungsgemäß außerordentlich bevorzugt sein, Mischungen der vorgenannten Öle einzusetzen.

Weitere erfindungsgemäß bevorzugte Ölkomponenten sind ausgewählt aus Siliconölen und Kohlenwasserstoffölen.

Erfindungsgemäß bevorzugte Siliconöle sind ausgewählt aus Dialkyl- und Alkylarylsiloxanen, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Weitere erfindungsgemäß bevorzugte Siliconöle sind ausgewählt aus flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind. Weitere erfindungsgemäß bevorzugte Siliconöle sind ausgewählt aus nichtflüchtigen höhermolekularen linearen Dimethylpolysiloxanen, im Handel erhältlich z. B. unter der Bezeichnung Dow Corning^{®} 190, Dow Corning^{®} 200 Fluid mit Viskositäten im Bereich von 5 - 100 cSt, bevorzugt 5 - 50 cSt oder auch 5 - 10 cSt, und Baysilon^{®} 350 M.

Erfindungsgemäß bevorzugte natürliche und synthetische Kohlenwasserstoffe sind ausgewählt aus Paraffinölen, Isohexadecan, Isoeicosan, Polyisobutenen und Polydecenen, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (Cetiol^{®}S).

Besonders bevorzugte erfindungsgemäße Deodorant- oder Antitranspirant-Zusammensetzungen sind dadurch gekennzeichnet, dass das/die bei 20 °C flüssige/n Öl/e in einer Gesamtmenge von 0,1 - 80 Gew.-%, bevorzugt 2 - 20 Gew.-%, besonders bevorzugt 3 - 15 Gew.-%, jeweils bezogen auf das Gesamtgewicht der Zusammensetzung, enthalten ist/sind.

In einer weiteren bevorzugten Ausführungsform der Erfindung weist ein Anteil der Ölkomponenten von mindestens 80 Gew.-% einen Brechungsindex n_{D} von 1,39 - 1,51 auf. Besonders bevorzugt ist es, wenn 5 - 40 - 50 Gew.-%, außerordentlich bevorzugt 10 - 12 - 25 - 30 Gew.-% der Ölkomponenten einen Brechungsindex n_{D} von 1,43 - 1,51, bevorzugt 1,44 - 1,49, besonders bevorzugt 1,45 - 1,47 - 1,485, bei 20 °C (gemessen bei λ = 589 nm) aufweisen.

Erfindungsgemäß besonders bevorzugte Zusammensetzungen enthalten weiterhin bevorzugt mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4- dioxaspiro (4.5)decan-2-methanol), Monomenthylsuccinat und 2-Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Erfindungsgemäß besonders bevorzugt ist, dass mindestens ein hautkühlender Wirkstoff in einer Gesamtmenge von 0,01 - 1 Gew.%, besonders bevorzugt 0,02 - 0,5 Gew.% und außerordentlich bevorzugt 0,05 - 0,2 Gew.%, jeweils bezogen auf das Gesamtgewicht der Zusammensetzung, enthalten ist.

Erfindungsgemäß besonders bevorzugte Zusammensetzungen, die als treibgasgetriebenes Aerosol konfektioniert sind, enthalten mindestens ein Treibmittel. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Die Menge der Treibmittel beträgt bevorzugt 20 - 80 Gew.%, besonders bevorzugt 30 - 70 Gew.% und außerordentlich bevorzugt 40 - 50 Gew.-%, jeweils bezogen auf das Gesamtgewicht der Zubereitung, bestehend aus der erfindungsgemäßen Zusammensetzung und dem Treibmittel.

Als Druckgasbehälter kommen Gefäße aus Metall (Aluminium, Weißblech, Zinn), geschütztem bzw. nicht-splitterndem Kunststoff oder aus Glas, das außen mit Kunststoff beschichtet ist, in Frage, bei deren Auswahl Druck- und Bruchfestigkeit, Korrosionsbeständigkeit, leichte Füllbarkeit wie auch ästhetische Gesichtspunkte, Handlichkeit, Bedruckbarkeit etc. eine Rolle spielen. Spezielle Innenschutzlacke gewährleisten die Korrosionsbeständigkeit gegenüber der erfindungsgemäßen Zusammensetzung.

### Polyole

Bevorzugte erfindungsgemäße Zusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind.

Bevorzugte erfindungsgemäße Deodorant- oder Antitranspirant-Zusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen.

Besonders bevorzugte erfindungsgemäße Deodorant- oder Antitranspirant-Zusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 30 Gew.-%, bevorzugt 8 - 25 Gew.-%, besonders bevorzugt 10 - 18 Gew.-%, jeweils bezogen auf die Gesamtzusammensetzung, enthalten ist.

Besonders bevorzugte erfindungsgemäße Deodorant- oder Antitranspirant-Zusammensetzungen sind dadurch gekennzeichnet, dass mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung stiftförmiger oder cremeförmiger Produkte und eine Minimierung der sichtbaren Rückstände auf der Haut. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol^{®} MM).

Weitere besonders bevorzugte erfindungsgemäße Deodorant- oder Antitranspirant-Zusammensetzungen, insbesondere solche in Stiftform, sind dadurch gekennzeichnet, dass die mindestens eine Lipid- oder Wachskomponente mit einem Schmelzpunkt im Bereich von 25 - < 50°C in Mengen von 0,01 bis 20 Gew.-%, bevorzugt 3 - 20 Gew.-%, besonders bevorzugt 5 - 18 Gew.-% und außerordentlich bevorzugt 6 - 15 Gew.-%, bezogen auf die Gesamtzusammensetzung, enthalten ist.

Besonders bevorzugte erfindungsgemäße Deodorant- oder Antitranspirant-Zusammensetzungen, die in Stiftform vorliegen, sind dadurch gekennzeichnet, dass sie zur Verbesserung der Stiftkonsistenz und der sensorischen Eigenschaften weiterhin mindestens einen festen, wasserunlöslichen teilchenförmigen Füllstoff enthalten, der von Talkum und Schichtsilicaten verschieden ist. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten vom Typ DRY FLO^{®}, Cellulose und Cellulosederivaten, Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Bornitrid, Lactoglobulinderivaten, z. B. Natrium-C₈₋₁₆-Isoalkyl-succinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich für den erfindungsgemäßen Zweck eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning).

Besonders bevorzugte erfindungsgemäße Deodorant- oder Antitranspirant-Zusammensetzungen sind dadurch gekennzeichnet, dass sie mindestens einen festen, wasserunlöslichen teilchenförmigen Füllstoff in einer Gesamtmenge von 0,01 bis 30 Gew.-%, bevorzugt 5 - 20 Gew.-%, besonders bevorzugt 8 - 15 Gew.-%, jeweils bezogen auf die Gesamtzusammensetzung, enthalten.

Besonders bevorzugte erfindungsgemäße Deodorant- oder Antitranspirant-Zusammensetzungen sind dadurch gekennzeichnet, dass weiterhin mindestens eine Duftstoffkomponente enthalten ist. Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höher siedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-betanaphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimatalkohol, Zimtsäure, Zimtsäureethylester, Zimtsäurebenzylester.

Zu den leichter flüchtigen Riechstoffen zählen insbesondere die niedriger siedenden Riechstoffe natürlichen oder synthetischen Ursprungs, die allein oder in Mischungen eingesetzt werden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Besonders bevorzugte erfindungsgemäße Deodorant- oder Antitranspirant-Zusammensetzungen sind dadurch gekennzeichnet, dass mindestens eine Duftstoffkomponente in einer Gesamtmenge von 0,00001 bis 4 Gew.-%, bevorzugt 0,5 - 2 Gew.-%, jeweils bezogen auf die Gesamtzusammensetzung, enthalten ist.

Weitere besonders bevorzugte erfindungsgemäße Deodorant- oder Antitranspirant-Zusammensetzungen sind dadurch gekennzeichnet, dass mindestens eine Duftstoffkomponente in verkapselter Form vorliegt. Eine bevorzugte Ausführungsform ist dadurch gekennzeichnet, dass das Kapselmaterial wasserlöslich ist. Ein bevorzugtes wasserlösliches Kapselmaterial ist Natriumstärkeoctenylsuccinat. Die Parfümkapseln setzen sich bevorzugt zusammen aus etwa 40% Natriumstärkeoctenylsuccinat, 10% Mannitol und ca. 50% Parfümöl.

Die Einsatzmenge an Parfümkapseln liegt bevorzugt im Bereich von 0,3 - 0,8 Gew.-%, bezogen auf die Gesamtmasse der Zubereitung. Darüber hinaus können bevorzugt 0,8 - 1,2 Gew.-% unverkapseltes, das heißt freies, Parfümöl, enthalten sein.

Vorteilhafte Bestandteile der erfindungsgemäßen Zubereitung sind Disteardimonium Hectorite, Aluminumchlorohydrat, Parfum und Silberlactat, vorteilhaft als Aerosol konfektioniert und vorteilhaft mit Butan und/oder Propan als Treibgase versetzt.

Ein weiterer Gegenstand der vorliegenden Anmeldung ist ein nicht-therapeutisches, kosmetisches Verfahren zur Reduzierung und/oder Regulierung der Schweißbildung und/oder des Körpergeruchs, bei dem eine erfindungsgemäße oder eine erfindungsgemäß bevorzugte Zusammensetzung, insbesondere eine Zusammensetzung gemäß einem der Ansprüche 1, 2, 3, 4, 5, 6, 7, 8, 9 oder 10 in einer wirksamen Menge auf die Haut, bevorzugt auf die Haut im Achselbereich, aufgetragen wird.

Bezüglich bevorzugter Ausführungsformen des erfindungsgemäßen Verfahrens gilt mutatis mutandis das zu den erfindungsgemäßen Zusammensetzungen Gesagte.

Ein weiterer Gegenstand der vorliegenden Anmeldung ist die nicht-therapeutische, kosmetische Verwendung einer erfindungsgemäßen oder einer erfindungsgemäß bevorzugten schweißhemmenden Zusammensetzung, insbesondere einer Zusammensetzung gemäß einem der Ansprüche 1, 2, 3, 4, 5, 6, 7, 8, 9 oder 10 zur Reduzierung und/oder Regulierung der Transpiration und/oder des Körpergeruchs.

Bezüglich bevorzugter Ausführungsformen der erfindungsgemäßen Verwendung gilt mutatis mutandis das zu den erfindungsgemäßen Zusammensetzungen Gesagte.

Die nachfolgenden Beispiele illustrieren die erfindungsgemäßen Zubereitungen, ohne sie hierauf zu beschränken. Die darin angeführten Anteile sind auf die Gesamtmasse der Zubereitung bzw. Wirkstofflösung bezogen.

Antitranspirantaerosole:

**Tabelle 1: Suspensionen zum Versprühen als Antitranspirant-Spray**

| | 1.1 | 1.2 | 1.3 | 1.4 | 1.5 | 1.6 |
|---|---|---|---|---|---|---|
| | | | | | | |
| Parfum | 5,00 | 5,00 | 5,00 | 5,00 | 5,00 | 5,00 |
| Aluminumchlorohydrat (aktiviert) | 30,00 | 35,00 | 35,00 | 35,00 | 28,00 | 32,00 |
| Parfümkapseln * | 2,00 | 2,00 | 2,00 | 1,50 | 1,50 | 1,00 |
| Silberlactat | 0,000913 | 0,000913 | 0,000913 | 0,000913 | 0,00183 | 0,00183 |
| Isopropylpalmitat | 5,00 | 5,00 | - | - | - | 6,00 |
| C12-15-Alkylbenzoat | -- | -- | 5,00 | 5,00 | -- | -- |
| Cosmacol PLG | - | - | - | - | 5,00 | -- |
| Disteardimonium Hectorite | 4,50 | 3,90 | 4,00 | 3,80 | 5,00 | 4,50 |
| Propylencarbonat | 1,50 | 1,30 | 1,30 | 1,30 | 1,70 | 1,50 |
| Cyclopentasiloxan | ad 100 | ad 100 | ad 100 | ad 100 | ad 100 | ad 100 |

Die schweißhemmenden Suspensionen 1.1 bis 1.6 wurden mit einem Butan/Isobutan/PropanGemisch als Treibgas im Abfüllverhältnis Suspension:Treibgas von 15:85 in einer Aerosoldose verpackt.

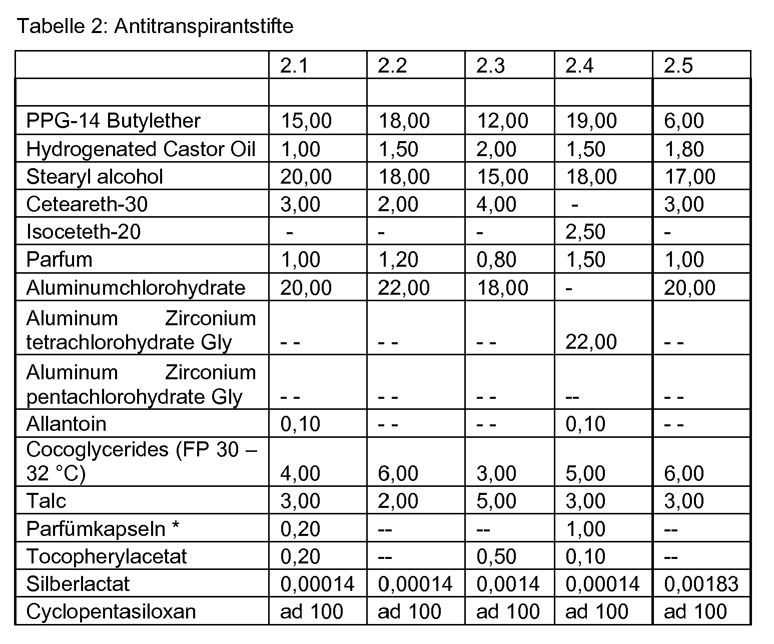

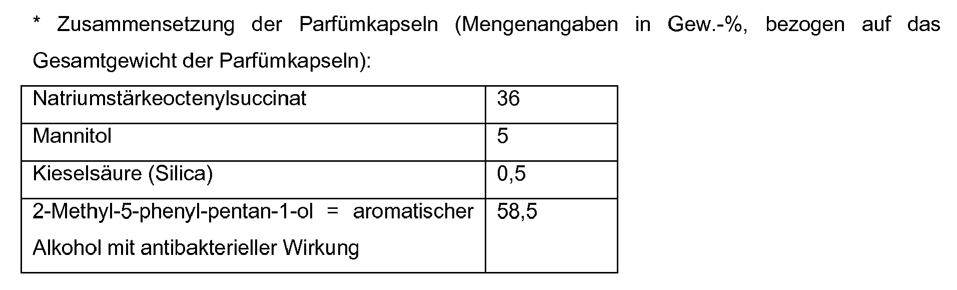

## Patentansprüche

1. Wasserfreie kosmetische oder dermatologische Zubereitung, enthaltend
a) Silberlactat in einer Gesamtmenge von 1,8 - 182 ppm,
b) ein oder mehrere Verdickungsmittel, ausgewählt aus der Gruppe der Schichtsilikate und/oder Talkum,
c) einen oder mehrere Antitranspirantwirkstoffe,
d) Parfum und
e) gegebenenfalls mindestens ein Treibmittel,
wobei zur Ermittlung der Gesamtmenge gegebenenfalls in der Zubereitung enthaltenes Treibmittel nicht berücksichtigt wird.

2. Zubereitung nach Anspruch 1, **dadurch gekennzeichnet, dass** der Antitranspirant-Wirkstoff ausgewählt ist aus den wasserlöslichen adstringierenden anorganischen und organischen Salzen des Aluminiums und des Zirkoniums sowie beliebigen Mischungen dieser Salze.

3. Zubereitung nach einem der vorstehenden Ansprüche, enthaltend aktiviertes Aluminiumchlorohydrat.

4. Zubereitung nach einem der vorstehenden Ansprüche, enthaltend verkapseltes Parfum, wobei das Kapselmaterial wasserlöslich ist.

5. Zubereitung nach einem der vorstehenden Ansprüche, **dadurch gekennzeichnet, dass** die Schichtsilikate ausgewählt sind aus Tonmineralien, insbesondere ausgewählt aus der Gruppe, bestehend aus Montmorrillonit, Nontronit, Hectorit, Saponit, Sauconit, Beidellit, Allevardit, Illit, Halloysit, Attapulgit und/oder Sepiolit.

6. Zubereitung nach einem der vorstehenden Ansprüche, enthaltend Disteardimonium Hectorit als Verdickungsmittel.

7. Zubereitung nach einem der vorstehenden Ansprüche als Aerosol, **dadurch gekennzeichnet, dass** Disteardimonium Hectorit, Aluminumchlorohydrat, Parfüm und Silberlactat enthalten sind.

8. Zubereitung nach einem der vorstehenden Ansprüche, **dadurch gekennzeichnet, dass** Silberlactat in einer Gesamtmenge von 3,6 - 91,3 ppm, bevorzugt 9,1 - 36,5 ppm, besonders bevorzugt 12,8 - 18,3 ppm, enthalten ist, wobei zur Ermittlung der Gesamtmenge gegebenenfalls in der Zubereitung enthaltenes Treibmittel nicht berücksichtigt wird.

9. Zubereitung nach einem der vorstehenden Ansprüche, **dadurch gekennzeichnet, dass** Silber in einer Gesamtmenge von 2 - 50 ppm, bevorzugt 5 - 20 ppm, besonders bevorzugt 7 - 10 ppm, enthalten ist, wobei zur Ermittlung der Gesamtmenge gegebenenfalls in der Zubereitung enthaltenes Treibmittel nicht berücksichtigt wird.

10. Zubereitung nach einem der vorstehenden Ansprüche, **dadurch gekennzeichnet, dass** das oder die Verdickungsmittel zu einem Anteil von 0,5 bis 10 Gew.-%, bevorzugt 1 - 7 Gew.-%, besonders bevorzugt 2 - 6 Gew.-%, außerordentlich bevorzugt 3 - 5 Gew.-%, enthalten sind, wobei zur Ermittlung des Anteils gegebenenfalls in der Zubereitung enthaltenes Treibmittel nicht berücksichtigt wird.

11. Nicht-therapeutisches, kosmetisches Verfahren zur Reduzierung und/oder Regulierung der Schweißbildung und/oder des Körpergeruchs, bei dem eine Zusammensetzung gemäß einem der Ansprüche 1, 2, 3, 4, 5, 6, 7, 8, 9 oder 10 in einer wirksamen Menge auf die Haut, bevorzugt auf die Haut im Achselbereich, aufgetragen wird.

12. Nicht-therapeutische, kosmetische Verwendung einer schweißhemmenden Zusammensetzung gemäß Anspruch 1, 2, 3, 4, 5, 6, 7, 8, 9 oder 10 zur Reduzierung und/oder Regulierung der Transpiration und/oder des Körpergeruchs.

## Claims

1. A water-free cosmetic or dermatological preparation, containing
a) silver lactate in a total amount of from 1.8 to 182 ppm,
b) one or more thickening agents, selected from the group of phyllosilicates and/or talc,
c) one or more antiperspirant active ingredients,
d) perfume, and
e) optionally at least one propellant,
wherein propellant optionally contained in the preparation is not taken into account for determining the total amount.

2. The preparation according to claim 1, **characterized in that** the antiperspirant active ingredient is selected from the water-soluble, astringent, inorganic and organic salts of aluminum and zirconium, and any mixtures of these salts.

3. The preparation according to one of the preceding claims, containing activated aluminum chlorohydrate.

4. The preparation according to one of the preceding claims, containing encapsulated perfume, wherein the capsule material is water soluble.

5. The preparation according to one of the preceding claims, **characterized in that** the phyllosilicates are selected from clay minerals, in particular selected from the group consisting of montmorillonite, nontronite, hectorite, saponite, sauconite, beidellite, allevardite, illite, halloysite, attapulgite and/or sepiolite.

6. The preparation according to one of the preceding claims, containing disteardimonium hectorite as a thickening agent.

7. The preparation according to one of the preceding claims in the form of an aerosol, **characterized in that** it contains disteardimonium hectorite, aluminium chlorohydrate, perfume and silver lactate.

8. The preparation according to one of the preceding claims, **characterized in that** silver lactate is contained in a total amount of from 3.6 to 91.3 ppm. preferably from 9.1 to 36.5 ppm, particularly preferably from 12.8 to 18.3 ppm, propellant optionally contained in the preparation not being taken into account for determining the total amount.

9. The preparation according to one of the preceding claims, **characterized in that** silver is contained in a total amount of from 2 to 50 ppm, preferably from 5 to 20 ppm, particularly preferably from 7 to 10 ppm, propellant optionally contained in the preparation not being taken into account for determining the total amount.

10. The preparation according to one of the preceding claims, **characterized in that** the thickening agent(s) is/are contained in a proportion of from 0.5 to 10 wt.%, preferably from 1 to 7 wt.%, particularly preferably from 2 to 6 wt.%, most preferably from 3 to 5 wt.%, propellant optionally contained in the preparation not being taken into account for determining the proportion.

11. A non-therapeutic, cosmetic method for reducing and/or regulating the formation of sweat and/or body odor, in which method a composition according to one of claims 1, 2, 3, 4, 5, 6, 7, 8, 9 or 10 is applied to the skin, preferably to the skin in the axilla region, in an effective amount.

12. Non-therapeutic, cosmetic use of an antiperspirant composition according to claim 1, 2, 3, 4, 5, 6, 7, 8, 9 or 10 for reducing and/or regulating perspiration and/or body odor.

## Revendications

1. Préparation cosmétique ou dermatologique anhydre contenant
a) du lactate d'argent dans une quantité totale de 1,8 à 182 ppm,
b) un ou plusieurs agents épaississants choisis dans le groupe des phyllosilicates et/ou du talc,
c) un ou plusieurs agents anti-transpirants,
d) des parfums et
e) éventuellement au moins un agent propulseur,
l'agent propulseur éventuellement présent dans la préparation n'étant pas pris en compte pour déterminer le montant total.

2. Préparation selon la revendication 1, **caractérisée en ce que** la substance active anti-transpirante est choisie parmi les sels minéraux et organiques astringents hydrosolubles de l'aluminium et du zirconium, ainsi que des mélanges quelconques de ces sels.

3. Préparation selon l'une des revendications précédentes, contenant du chlorhydrate d'aluminium activé.

4. Préparation selon l'une des revendications précédentes, contenant un parfum encapsulé, le matériau d'encapsulage est hydrosoluble.

5. Préparation selon l'une quelconque des revendications précédentes, **caractérisée en ce que** les phyllosilicates sont choisis parmi les minéraux argileux, en particulier choisis dans le groupe comportant la montmorillonite, la nontronite, l'hectorite, la saponite, la sauconite, la beidellite, l'allevardite, l'illite, l'halloysite, l'attapulgite, et/ou la sépiolite.

6. Préparation selon l'une des revendications précédentes, comprenant du disteardimonium hectorite comme épaississant.

7. Préparation selon l'une des revendications précédentes, utilisée en aérosol, **caractérisée en ce qu'**elle contient du disteardimonium hectorite, du chlorhydrate d'aluminium, un parfum et du lactate d'argent.

8. Préparation selon l'une des revendications précédentes, **caractérisé en ce que** le lactate d'argent est contenu dans une quantité totale de 3,6 à 91,3 ppm, de préférence de 9,1 à 36,5 ppm, notamment de 12,8 à 18,3 ppm, l'agent propulseur éventuellement présent dans la préparation n'étant pas pris en compte pour déterminer le montant total.

9. Préparation selon l'une des revendications précédentes, **caractérisée en ce que** l'argent est contenu dans une quantité totale de 2 à 50 ppm, de préférence de 5 à 20 ppm, de manière particulièrement préférée de 7 à 10 ppm, l'agent propulseur éventuellement présent dans la préparation n'étant pas pris en compte pour déterminer le montant total.

10. Préparation selon l'une des revendications précédentes, **caractérisée en ce que** le ou les agents épaississants sont contenus dans une quantité de 0,5 à 10% en poids, de préférence de 1 à 7% en poids, de manière particulièrement préférée de 2 à 6% en poids, de manière extraordinairement préférée de 3 à 5% en poids, l'agent propulseur éventuellement présent dans la préparation n'étant pas pris en compte pour déterminer le montant total.

11. Procédé cosmétique non thérapeutique destiné à réduire et/ou réguler la transpiration et/ou l'odeur corporelle, dans lequel une composition selon l'une des revendications 1, 2, 3, 4, 5, 6, 7, 8, 9 ou 10 est appliquée dans une quantité efficace sur la peau, de préférence sur la peau dans la région des aisselles.

12. Utilisation cosmétique non thérapeutique d'une composition anti-transpirante selon les revendications 1, 2, 3, 4, 5, 6, 7, 8, 9 ou 10 pour diminuer et/ou réguler la transpiration et/ou l'odeur corporelle.
